# EUROPEAN PATENT APPLICATION

(11) **EP 3 957 755 A1**
(43) Date of publication of application: **23.02.2022**
(21) Application number: 21772078.8
(22) Date of filing: 24.02.2021
(51) Int. Cl.: C12Q 1/70, C12Q 1/6804, C12N 15/11

(54) **NOVEL CORONAVIRUS NUCLEIC ACID RAPID HYBRIDIZATION CAPTURE IMMUNOFLUORESCENCE DETECTION KIT, AND PREPARATION METHOD AND DETECTION METHOD**

(30) Priority: 20.03.2020 CN 202010199077
(71) Applicant: Anbio (Xiamen) Biotechnology Co., Ltd., Xiamen, Fujian 361000 (CN)
(72) Inventor: LEI, Yang, Xiamen, Fujian 361000 (CN); ZHANG, Jieli, Xiamen, Fujian 361000 (CN); ZHANG, Liwei, Xiamen, Fujian 361000 (CN); HUANG, Renxun, Xiamen, Fujian 361000 (CN); BAI, Jiawei, Xiamen, Fujian 361000 (CN); LE, Yicui, Xiamen, Fujian 36100 (CN); HU, Xiao, Xiamen, Fujian 361000 (CN); WANG, Daming, Xiamen, Fujian 361000 (CN)
(74) Representative: Loo, Chi Ching
(86) International application number: PCT/CN2021/077652
(87) International publication number: WO 2021/185034

(57) **Abstract**

The invention relates to the technical field of nucleic acid detection and discloses a 2019 novel coronavirus nucleic acid rapid hybrid capture immunofluorescence detection kit, and a preparation method and a detection method thereof. The kit includes COVID-19 reaction liquid, wherein the COVID-19 reaction liquid is prepared from a COVID-19 fluorescence marker and a COVID-19 probe solution; and the COVID-19 probe solution includes: an ORF1ab segment probe, an N segment probe and an E segment probe. Compared with general fluorescence PCR and sequencing detection, the kit has the advantages of stronger signal intensity, better specificity, shorter detection time, no need of professional technicians for operation, no need of refrigeration in transportation and storage, no need of a matched laboratory and a matched PCR instrument, and convenience and rapidness in use.

## Description

### BACKGROUND OF THE INVENTION

### 1. Technical Field

The invention relates to the technical field of nucleic acid detection, and more particularly, to a 2019 novel coronavirus nucleic acid rapid hybrid capture immunofluorescence detection kit, and a preparation method thereof.

### 2. Description of Related Art

Corona virus disease 2019 (COVID-19) belongs to β coronavirus, which has an envelope, round or elliptical particles and a diameter of 60-140 and is often polymorphous. The gene characteristics of the COVID-19 are obviously different from those of SARSr-CoV and MERSr-CoV, and the homology with bat SARS-like coronavirus (bat-SL-CoVZC45) is more than 85%.

The pneumonia infected by COVID-19 is lung inflammation caused by COVID-19, which has been incorporated into category B infectious disease stipulated in *Law of the People's Republic of China on Prevention and Control of Infectious Diseases,* and measures for prevention and control of category A infectious disease has been taken. With the spread of the epidemic, there is an urgent need for a detection reagent capable of accurately and rapidly diagnosing COVID-19.

At present, six enterprises have obtained the registration number of COVID-19 nucleic acid detection reagent:
1) COVID-19 nucleic acid detection kit (fluorescence PCR) of Sansure Biotech Co., Ltd. (GXZZ20203400064);
2) COVID-19 nucleic acid detection kit (fluorescence PCR) of Sun Yat-sen University Daan Gene Co., Ltd. (GXZZ20203400063);
3) COVID-19 nucleic acid detection kit (fluorescence PCR) of Shanghai GenenoDx Biotech Co., Ltd. (GXZZ20203400058);
4) COVID-19 nucleic acid detection kit (fluorescence PCR) of Shanghai Liferiver Biotech Co., Ltd. (GXZZ20203400057);
5) COVID-19 nucleic acid detection kit (fluorescence PCR) of BGI Biotech (Wuhan) Co., Ltd. (GXZZ20203400060); and
6) COVID-19 nucleic acid detection kit (combinatorial probe-anchor synthesis) of BGI Biotech (Wuhan) Co., Ltd. (GXZZ20203400059).

The above COVID-19 nucleic acid detection kits mainly adopt a fluorescence PCR nucleic acid detection method. The detection principle is that a fluorescent reporter group and a fluorescent quenching group are added into a PCR reaction system, amplification products accumulate continuously with the progress of the PCR reaction, which leads to the continuous accumulation of fluorescent signals, the fluorescent signals are monitored in real time and threshold cycle (Ct) values of unknown samples are obtained, and the Ct value refers to the minimum cycle number required for generating detectable fluorescent signals and is the cycle number corresponding to an inflection point where the fluorescent signals enter an exponential growth stage from background in the PCR cyclic process. The Ct values of standard samples with different concentrations are usually used to generate a standard curve, and an initial template amount of the unknown sample is calculated automatically from the standard curve.

The fluorescence PCR nucleic acid detection method is accurate in quantification and high in reproducibility, but the method needs professional PCR laboratories, special PCR instruments, specially trained technicians for operation, nucleic acid extraction and purification for samples and cold chain transportation below -20°C, has detection time as long as 1-3 hours, low detection efficiency and nucleic acid amplification in the detection process, is liable to cause laboratory positive product pollution and many other problems, and is inconvenient to use.

### BRIEF SUMMARY OF THE INVENTION

In order to solve the above problems, the invention provides a 2019 novel coronavirus nucleic acid rapid hybrid capture immunofluorescence detection kit, and a preparation method and a detection method thereof, which is shorter in detection time, does not need professional technicians for operation and matched laboratories and special instruments, and is more convenient and rapid in use.

A first objective of the invention is to provide a corona virus disease 2019 nucleic acid rapid hybrid capture immunofluorescence detection kit. The kit includes COVID-19 reaction liquid, wherein the COVID-19 reaction liquid is prepared from a COVID-19 fluorescence marker and a COVID-19 probe solution; the COVID-19 probe solution includes: an ORF1ab segment probe, an N segment probe and an E segment probe; and the ORF1ab segment probe is used for detecting an open reading coding frame lab of the COVID-19, the N segment probe is used for detecting an envelope protein gene of the COVID-19, and the E segment probe is used for detecting a core-shell protein gene of the COVID-19.

Preferably, a sequence of the ORF1ab segment probe is: 
Aacgattgtgcatcagctgactgaagcatgggttcgcggagttgatcacaactacagccataacctttccac
ataccgcagac;
 a sequence of the N segment probe is:
Agagcagcatcaccgccattgccagccattctagcaggagaagttc; and
a sequence of the E segment probe is:
aaggatggctagtgtaactagcaagaataccacgaaagcaagaaaaa.

Preferably, the COVID-19 fluorescence marker is prepared by coupling a fluorescent material and a COVID-19 marking raw material; the COVID-19 marking raw material adopts a COVID-19 antigen or antibody; and the fluorescent material adopts any one of the followings: a FITC fluorescein, a fluorescent microsphere, a fluorescent particle and a biological fluorescein.

Preferably, the kit further includes a COVID-19 negative reference substance, wherein the negative reference substance includes one or more of the following reference substances: a normal saline reference substance, a purified water reference substance, a non-COVID-19 pathogen reference substance and a pseudovirus not containing a COVID-19 target sequence.

Further, the negative reference substance includes N1-N17: N1-N2 are normal saline reference substances, N3-N4 are purified water reference substances, N5-N13 are human throat swab samples and N14-N17 are pseudoviruses not containing COVID-19 target sequences; in the human throat swab samples, COVID-19 is negative and the non-COVID-19 pathogen reference substance is positive; and the pseudoviruses not containing the COVID-19 target sequences are subtypes of coronaviruses, wherein N14 is positive for a human coronavirus 229E N segment, N15 is positive for a human coronavirus NL63 N segment, N16 is positive for a human coronavirus OC43 N segment, and N17 is positive for a human coronavirus HKU1 N segment.

Preferably, the kit further includes a COVID-19 positive reference substance, wherein the positive reference substance is a pseudovirus containing a COVID-19 target sequence; the positive control reference substance includes P1, P2 and P3, P1 being positive for a COVID-19 N segment, P2 being positive for a COVID-19 E segment, and P3 being positive for a COVID-19 ORF 1ab segment; and a concentration of the positive reference substance is 3000 TU/mL±5%.

Preferably, the kith further includes a precision reference substance and a limit of detection reference substance, wherein the precision reference substance includes J1, J2 and J3, J1-J2 being pseudoviruses containing COVID-19 target sequences and being positive for a COVID-19 ORF lab segment, concentrations of J1-J2 being 2000TU/mL±5% and 5000TU/mL±5% respectively, J3 being a mixed human negative throat swab sample and being negative for COVID-19; and the limit of detection reference substance includes L1, L2 and L3, L1 being positive for a COVID-19 N segment, L2 being positive for a COVID-19 E segment, L3 being positive for a COVID-19 ORF lab segment, and a concentration of the limit of detection reference substance being 1000 TU/mL±5%.

Preferably, the kit further includes a COVID-19 detection strip, COVID-19 redissolving liquid and COVID-19 sample preserving liquid.

A second objective of the invention is to provide a preparation method of any one of the above corona virus disease 2019 nucleic acid rapid hybrid capture immunofluorescence detection kits. The preparation method includes: a preparation step of the COVID-19 fluorescence marker as follows:
activation: adding 1% fluorescent microsphere solution with a ratio of 1.0mg/mL±5% and an EDC solution with a ratio of 0.6mg/mL±5% into a prepared borate buffer solution of 0.05M±5%, mixing uniformly, placing the mixture on a rotary mixer to rotate for more than 20 minutes, perform centrifugation at 15000-16000rpm for more than 30 minutes after activating, removing a supernatant, performing resuspension by the borate buffer solution of 0.05M±5% and mixing uniformly;
coupling: adding a COVID-19 antibody in an amount of 0.2mg/mL into the activated fluorescent microsphere solution, mixing uniformly, and placing the mixture on the rotary mixer to rotate for more than 2 hours to obtain a fluorescent microsphere marking conjugate solution;
closing: adding a 10% BSA solution with a ratio of 0.1ml/mL±5% into the fluorescent microsphere marking conjugate solution, mixing uniformly, and placing the mixture on the rotary mixer to rotate for 12-16 hours; and
centrifugal resuspension: centrifuging the fluorescent microsphere marking conjugate solution at 15000-16000rpm, removing a supernatant, washing with an isovolumetric borate buffer solution of 0.05M±5%, and finally resuspending a precipitate with a marker diluent in a volume which is equal to that of the supernatant solution to prepare the COVID-19 fluorescence marker.

Preferably, the preparation method includes a preparation step of COVID-19 reaction liquid as follows:
preparation of a fluorescence marker: diluting the COVID-19 fluorescence marker with a marker diluent according to a ratio, wherein the ratio of the diluent to a T line marker to a C line marker is equal to 17:2:1;
preparation of a probe solution: diluting a COVID-19 probe with nucleic acid solving liquid to 10µM±5% to obtain a COVID-19 probe solution;
preparation of reaction liquid: mixing the diluted COVID-19 fluorescence marker and the COVID-19 probe solution according to a volume ratio of 1:1 to obtain COVID-19 reaction liquid; and
subpackaging of the reaction liquid: subpackaging the COVID-19 reaction liquid into reaction tubes and drying for 6 to 8 hours under the conditions that the temperature is 18-28°C and the humidity is less than or equal to 30%.

Preferably, the preparation method further includes a preparation step of a COVID-19 positive reference substance: diluting artificially synthesized COVID-19 RNA with a positive reference substance diluent to 2000TU/mL±5%; and subpackaging the diluted positive reference substances into vertical tubes and drying for 6 to 8 hours under the conditions that the temperature is 18-28°C and the humidity is less than or equal to 30%.

A third objective of the invention is to provide a detection method of any one of the above corona virus disease 2019 nucleic acid rapid hybrid capture immunofluorescence detection kits. The detection method includes the following steps:
acquiring a target nucleic acid fragment in a to-be-detected sample through hybrid capture;
performing nucleic acid hybridization reaction on the target nucleic acid fragment and the COVID-19 reaction liquid to obtain to-be-detected liquid; and
adding the to-be-detected liquid into a COVID-19 detection strip for fluorescence signal recognition.

The beneficial effects of the invention are:
the COVID-19 nucleic acid rapid hybrid capture immunofluorescence detection kit provided by the invention adopts a hybrid capture-immunofluorescence analysis (short for HC-IFA), which is a method for acquiring target nucleic acid fragments in samples through hybrid capture and qualitatively, semi-quantificationally and quantificationally judging the number of the target nucleic acid fragments in the samples through fluorescent signal recognition. Compared with general antigen-antibody immunodetection, the invention covers nucleic acid detection, achieves detection sensitivity at molecular level and realizes fluorescence recognition of nucleic acid detection. Compared with general fluorescence PCR and sequencing detection, the kit has the advantages of stronger signal intensity, better specificity, shorter detection time, no need of professional technicians for operation, no need of refrigeration in transportation and storage, no temperature changing link in a reaction process and no need of a matched laboratory and a matched PCR instrument, can perform single sample detection with a detection time of 30 minutes, has short detection tie, and has a detection flux of 100 to 200 copies per hour.

### BRIEF DESCRIPTION OF THE SEVERAL VIEWS OF THE DRAWINGS

The accompanying drawings described here are provided for further understanding of the invention, and constitute a part of the invention. The exemplary embodiments and illustrations of the invention are intended to explain the invention, but do not constitute inappropriate limitations to the invention. In the accompanying drawings:
FIG. 1 is a flowchart of a preparation process of a corona virus disease 2019 nucleic acid rapid hybrid capture immunofluorescence detection kit.
FIG. 2 is S9.6 protein model information used in the specific detection process of a corona virus disease 2019 nucleic acid rapid hybrid capture immunofluorescence detection kit.

### DETAILED DESCRIPTION OF THE INVENTION

In order to make the technical problems to be solved, technical solutions and beneficial effects of the invention more apparent, the invention will be described in more detail with reference to the drawings and embodiments. It should be understood that the specific embodiments described herein are merely intended to explain the invention, rather than to limit the invention.

### First embodiment (kit):

The embodiment provides a 2019 novel coronavirus nucleic acid rapid hybrid capture immunofluorescence detection kit. The kit includes COVID-19 reaction liquid, COVID-19 redissolving liquid, a COVID-19 negative reference substance and COVID-19 sample preserving liquid which are subpackaged into reaction tubes or vertical tubes respectively, and further includes a COVID-19 detection strip, an aluminum foil bag card, a kit label, a kit instruction and the like.

The COVID-19 reaction liquid is prepared from a COVID-19 fluorescence marker and a COVID-19 probe solution; and the COVID-19 probe solution includes: an ORF1ab segment probe, an N segment probe and an E segment probe; wherein the ORF1ab segment probe is used for detecting an open reading coding frame lab of the COVID-19, the N segment probe is used for detecting an envelope protein gene of the COVID-19, and the E segment probe is used for detecting a core-shell protein gene of the COVID-19.

A sequence of the ORF1ab segment probe is: 
Aacgattgtgcatcagctgactgaagcatgggttcgcggagttgatcacaactacagccataacctttccac
ataccgcagac;
 a sequence of the N segment probe is:
Agagcagcatcaccgccattgccagccattctagcaggagaagttc; and
a sequence of the E segment probe is:
aaggatggctagtgtaactagcaagaataccacgaaagcaagaaaaa.

The COVID-19 fluorescence marker is prepared by coupling a fluorescent material and a COVID-19 marking raw material; the COVID-19 marking raw material adopts a COVID-19 antibody; and the fluorescent material adopts any one of the followings: a FITC fluorescein, a fluorescent microsphere, a fluorescent particle, a biological fluorescein and other substances capable of emitting fluorescence, but is not limited to this.

The COVID-19 negative reference substance includes one or more of the following reference substances: a normal saline reference substance, a purified water reference substance, a non-COVID-19 pathogen reference substance and a pseudovirus not containing a COVID-19 target sequence. Specifically, the negative reference substance includes N1-N17: N1-N2 are normal saline reference substances, N3-N4 are purified water reference substances, N5-N13 are human throat swab samples and N14-N17 are pseudoviruses not containing COVID-19 target sequences; in the human throat swab samples, COVID-19 is negative and the non-COVID-19 pathogen reference substance is positive; and the pseudoviruses not containing the COVID-19 target sequences are subtypes of coronaviruses, wherein N14 is positive for a human coronavirus 229E N segment, N15 is positive for a human coronavirus NL63 N segment, N16 is positive for a human coronavirus OC43 N segment, and N17 is positive for a human coronavirus HKU1 N segment.

The COVID-19 positive reference substance is a pseudovirus containing a COVID-19 target sequence; the positive control reference substance includes P1, P2 and P3, wherein P1 is positive for a COVID-19 N segment, P2 is positive for a COVID-19 E segment, and P3 is positive for a COVID-19 ORF 1ab segment; and a concentration of the positive reference substance is 3000 TU/mL±5%.

The precision reference substance includes J1, J2 and J3, wherein J1-J2 are pseudoviruses containing COVID-19 target sequences and are positive for a COVID-19 ORF lab segment, concentrations of J1-J2 are 2000TU/mL±5% and 5000TU/mL±5% respectively, J3 is a mixed human negative throat swab sample and is negative for COVID-19; and the limit of detection reference substance includes L1, L2 and L3, wherein L1 is positive for a COVID-19 N segment, L2 is positive for a COVID-19 E segment, L3 is positive for a COVID-19 ORF lab segment, and a concentration of the limit of detection reference substance is 1000 TU/mL± 5. The detail is shown in Table 1 below.

**Table 1 Composition table of reference products**

| Type of Reference Product | Serial Number | Information | Sample Type | Concentration |
|---|---|---|---|---|
| Positive reference | P1 | Positive for COVID-19 N segment | Pseudovirus | 3000 TU/mL |
| product | P2 | Positive for COVID-19 E segment | Pseudovirus | 3000 TU/mL |
| | P3 | Positive for COVID-19 ORF 1ab segment | Pseudovirus | 3000 TU/mL |
| Negative reference product | N1 | Normal saline | / | / |
| | N2 | Normal saline | / | / |
| | N3 | Purified water | / | / |
| | N4 | Purified water | / | / |
| | N5 | Negative for COVID-19 Positive for influenza A virus | Throat swab liquid | / |
| | N6 | Negative for COVID-19 Positive for metapneumovirus | Throat swab liquid | / |
| | N7 | Negative for COVID-19 Positive for influenza B virus | Throat swab liquid | / |
| | N8 | Negative for COVID-19 Positive for parainfluenza virus | Throat swab liquid | / |
| | N9 | Negative for COVID-19 Positive for respiratory syncytial virus | Throat swab liquid | / |
| | N10 | Negative for COVID-19 Positive for rhinovirus | Throat swab liquid | / |
| | N11 | Negative for COVID-19 Positive for adenovirus | Throat swab liquid | / |
| | N12 | Negative for COVID-19 Positive for mycoplasma | Throat swab liquid | / |
| | | pneumoniae | | |
| | N13 | Negative for COVID-19 Positive for chlamydia pneumoniae | Throat swab liquid | / |
| | N14 | Negative for COVID-19 Positive for human coronavirus 229E N segment | Pseudovirus | / |
| | N15 | Negative for COVID-19 Positive for human coronavirus NL63 N segment | Pseudovirus | / |
| | N16 | Negative for COVID-19 Positive for human coronavirus OC43 N segment | Pseudovirus | / |
| | N17 | Negative for COVID-19 Positive for human coronavirus HKU1 N segment | Pseudovirus | / |
| Detection limit reference product | L1 | Positive for COVID-19 N segment | Pseudovirus | 1000 TU/mL |
| | L2 | Positive for COVID-19 E segment | Pseudovirus | 1000 TU/mL |
| | L3 | Positive for COVID-19 ORF 1ab segment | Pseudovirus | 1000 TU/mL |
| Precision reference product | J1 | Positive for COVID-19 ORF 1ab segment | Pseudovirus | 2000 TU/mL |
| | J2 | Positive for COVID-19 ORF | Pseudovirus | 5000 TU/mL |
| | | 1ab segment | | |
| | J3 | Negative for COVID-19 | Throat swab liquid | / |

The detection strip may adopt a chromatography or percolation method. In this embodiment, the detection strip adopts an immunofluorescence chromatography test strip, which includes a water-absorbing pad arranged on an end area of the detection strip, a sample adding area arranged on the other end area of the detection strip, and a nitrocellulose membrane (NC membrane) arranged between the water-absorbing pad and the sample adding area, wherein the nitrocellulose membrane is arranged on a T line detection area. The treated glass fiber pad, the treated nitrocellulose membrane and the water-absorbing pad are sequentially in lap joint with a PVC bottom plate and are cut into test strips with a set width, that is, the detection strip of the invention.

### Second embodiment (preparation method):

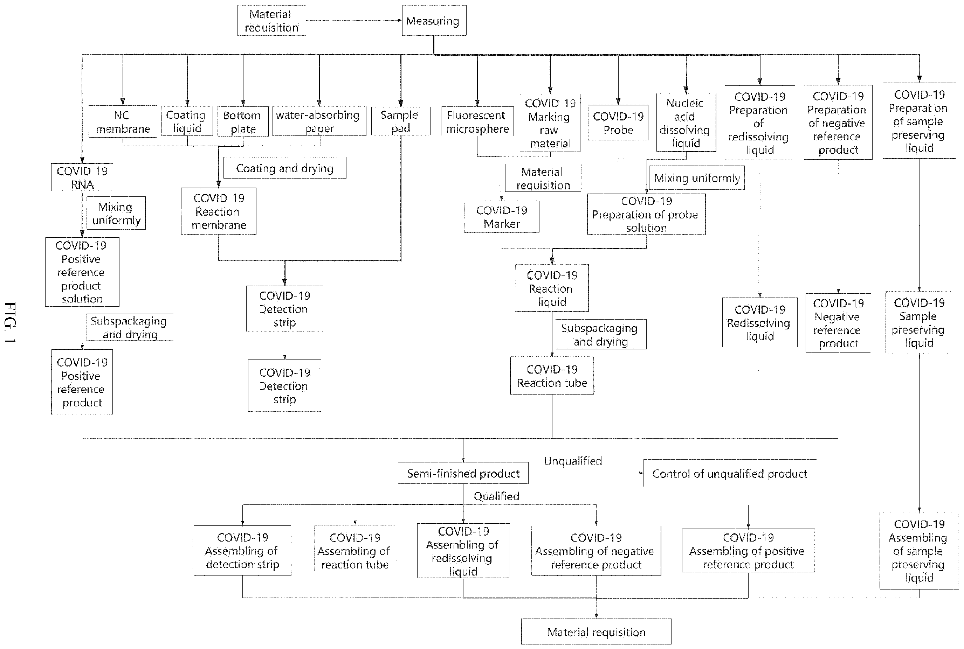

As shown in FIG. 1, the embodiment provides a preparation method of any one of the above corona virus disease 2019 nucleic acid rapid hybrid capture immunofluorescence detection kits. The preparation method includes:
(1) a preparation step of a COVID-19 fluorescence marker as follows:
   activation: 1% fluorescent microsphere solution with a ratio of 1.0mg/mL±5% and an EDC solution with a ratio of 0.6mg/mL±5% were added into a prepared borate buffer solution of 0.05M±5% for uniformly mixing, the mixture was placed on a rotary mixer to rotate for more than 20 minutes, centrifugation was performed at 15000-16000rpm for more than 30 minutes after activating, a supernatant was removed, resuspension was performed by the borate buffer solution of 0.05M±5% and uniform mixing was performed;
   coupling: a COVID-19 antibody in an amount of 0.2mg/mL±5% was added into the activated fluorescent microsphere solution for uniform mixing, and the mixture was placed on the rotary mixer to rotate for more than 2 hours to obtain a fluorescent microsphere marking conjugate solution;
   closing: a 10% BSA solution with a ratio of 0.1ml/mL±5% was added into the fluorescent microsphere marking conjugate solution for uniform mixing, and the mixture was placed on the rotary mixer to rotate for 12-16 hours; and
   centrifugal resuspension: the fluorescent microsphere marking conjugate solution was centrifuged at 15000-16000rpm, a supernatant was removed, the material was washed with an isovolumetric borate buffer solution of 0.05M±5%, and finally a precipitate was resuspended with a marker diluent in a volume which is equal to that of the supernatant solution to prepare the COVID-19 fluorescence marker.
(2) A preparation step of COVID-19 reaction liquid as follows:
   preparation of a fluorescence marker: the COVID-19 fluorescence marker was diluted with a marker diluent according to a ratio, wherein the ratio of the diluent to a T line marker to a C line marker is equal to 17:2:1;
   preparation of a probe solution: a COVID-19 probe was diluted with nucleic acid solving liquid to 10µM±5% to obtain a COVID-19 probe solution;
   preparation of reaction liquid: the diluted COVID-19 fluorescence marker and the COVID-19 probe solution were mixed according to a volume ratio of 1:1 to obtain COVID-19 reaction liquid; and
   subpackaging of the reaction liquid: the COVID-19 reaction liquid was subpackaged into reaction tubes (4µL/tube) and dried for 6 to 8 hours under the conditions that the temperature is 18-28°C and the humidity is less than or equal to 30%.
(3) A preparation step of a COVID-19 positive reference substance: artificially synthesized COVID-19 RNA was diluted with a positive reference substance diluent to 2000TU/mL±5%; and the diluted positive reference substances were subpackaged into vertical tubes (5µL/tube) and dried for 6 to 8 hours under the conditions that the temperature is 18-28°C and the humidity is less than or equal to 30%.
(4) Coating conditions of the COVID-19 detection strip:
   the T line coating concentration is 0.5mg/mL±5% and the C line coating concentration is 1.0mg/mL±5%;
   the spray amount is 1.0µL/cm±5%, the speed of a guide rail is 100mm/s±5%, and nozzle interval is 6mm±5%; and
   drying: temperature is 18-28°C and humidity is less than or equal to 30%, and drying time:16h-20h.
(5) Detection conditions of the detection reagent:
   the adding amount of the reaction tube redissolving liquid is 85µL±5%;
   the sampling amount of the sample is 20µL±5%;
   the uniformly mixed sample of 100µL±5% was absorbed and added into a sample hole of a detection card;
   the nucleic acid hybridization reaction condition is 37°C and the reaction time is more than 15 minutes; and
   the reagent detection reaction time is more than 15 minutes.
(6) Freeze-drying process:
   intermediate preparations involved in the reaction process of the preparation method of the embodiment are all freeze-dried products, and it is only necessary to prepare working liquid without complicated operation such as dilution; and the freeze-drying processing method is: freeze drying was performed at -30°C for 5 hours, the temperature was gradually increased to -10°C in the subsequent 12-hour freeze-drying process at the heating rate of 5°C/3h, and finally freeze drying was performed at -10°C for 7 to 19 hours.

Third embodiment (detection method):

This embodiment further provides a detection method of any one of the above corona virus disease 2019 nucleic acid rapid hybrid capture immunofluorescence detection kits. The detection method includes the following steps:
a target nucleic acid segment in a to-be-detected sample was acquired through hybrid capture;
performing nucleic acid hybridization reaction on the target nucleic acid fragment and the COVID-19 reaction liquid to obtain to-be-detected liquid; and
the to-be-detected liquid was added into the COVID-19 detection strip for fluorescent signal recognition. In this embodiment, the to-be-detected liquid was added into a sample adding area of the detection strip, 100ul of washing liquid was added again from the sample adding area after 90 seconds, and after 10 minutes, a detection area of the detection strip was subjected to fluorescence detection to read the detection result.

The detection result was judged as follows:
irradiation was performed by using 480nm exciting light to recognize a 520nm fluorescence emitting signal, and if there is the fluorescence emitting signal, it is interpreted as positive, otherwise, it is interpreted as negative. The intensity of reaction may be interpreted according to the intensity of the emitting signal as required, and the recognition instrument may adopt a general fluorescence reading instrument, for example, a portable immunofluorescence analyzer produced by Suzhou Hemai Science and Technology.

It should be noted that the embodiments in this specification are described in a progressive manner. Each embodiment focuses on a difference from other embodiments. The same or similar part of the embodiments may be referenced to each other. The preparation method and detection method embodiments are basically similar to the kit embodiments, so the description is relatively simple, and the relevant points are referenced to the partial description of the kit embodiments.

The effect comparison of an existing fluorescence PCR nucleic acid detection method and the corona virus disease 2019 nucleic acid rapid hybrid capture immunofluorescence detection kit based on a nucleic acid hybrid capture immunofluorescence analysis method and the detection method thereof is specifically as follows:

**Table 2 Technical effect comparison diagram**

| Comparison Item | Invention | Prior Art |
|---|---|---|
| Methodological principle | Nucleic acid hybrid capture fluorescence method | Nucleic acid amplification (fluorescence PCR method) |
| Sample treatment | Free of nucleic acid extraction | Nucleic acid extraction and purification |
| Detection time | 30 minutes | 1 to 3 hours |
| Usage environment | On-site detection and on-demand detection are realized without a PCR laboratory | Three professional PCR laboratories and a large central laboratory are required; and if polluted, they cannot be used for a long time |
| Staff requirement | No need for professional technicians, and personnel can be simply trained | Specially trained technicians are required |
| Device | Matched device, which is small, light and portable | The PCR instrument is required and cannot swing |
| Application Scene | Primary medical care, outpatient service, clinical laboratory, emergency treatment, exit and entry, customs, center for | Central laboratories of large Grade III Level A hospitals |

| | | |
|---|---|---|
| | disease control and prevention, and the like | |
| Potential pollution | No amplification, no pollution of laboratory positive products, and the redissolving liquid has an "anti-virus" component | Nucleic acid amplification, liable to cause pollution of laboratory positive products, and the pollution cannot be eliminated for a long time |
| Transportation and storage | Storage at normal temperature of 2-30°C and transportation at normal temperature | Storage at -20°C, and cold chain transportation is required |

The specific detection process of the invention is described as follows:
(1) a throat swab sample was used and put into a sample preserving tube (the sample preserving tube contains preserving liquid), a mixture of the preserving liquid and the sample was obtained, and the mixture was defined as a detection sample.
(2) A proper amount of detection sample was put into a reaction tube, wherein the reaction tube contains powder after reaction liquid is dried and the power is defined as reaction liquid powder. The preparation of the reaction liquid powder may be referenced to the followings:
   preparation of a probe solution: a COVID-19 probe was diluted with nucleic acid solving liquid to 10µM±5% to obtain a COVID-19 probe solution; preparation of reaction liquid: the diluted COVID-19 fluorescence marker and the COVID-19 probe solution were mixed according to a volume ratio of 1:1 to obtain COVID-19 reaction liquid; and subpackaging of the reaction liquid: the COVID-19 reaction liquid was subpackaged into reaction tubes (4µL/tube) and dried for 6 to 8 hours under the conditions that the temperature is 18-28°C and the humidity is less than or equal to 30%. The reaction liquid powder contains the probe solution and protein marked with a fluorescence signal. The preparation process of the protein marked with the fluorescence signal may be referenced to the followings:
   activation: 1% fluorescent microsphere solution with a ratio of 1.0mg/mL±5% and an EDC solution with a ratio of 0.6mg/mL±5% were added into a prepared borate buffer solution of 0.05M±5% for uniformly mixing, the mixture was placed on a rotary mixer to rotate for more than 20 minutes, centrifugation was performed at 15000-16000rpm for more than 30 minutes after activating, a supernatant was removed, resuspension was performed by the borate buffer solution of 0.05M±5% and uniform mixing was performed.
   coupling: a COVID-19 antibody in an amount of 0.2mg/mL was added into the activated fluorescent microsphere solution for uniform mixing, and the mixture was placed on the rotary mixer to rotate for more than 2 hours to obtain a fluorescent microsphere marking conjugate solution.
   closing: a 10% BSA solution with a ratio of 0.1ml/mL±5% was added into the fluorescent microsphere marking conjugate solution for uniform mixing, and the mixture was placed on the rotary mixer to rotate for 12-16 hours.
   centrifugal resuspension: the fluorescent microsphere marking conjugate solution was centrifuged at 15000-16000rpm, a supernatant was removed, the material was washed with an isovolumetric borate buffer solution (PH 8.0) of 0.05M±5%, and finally a precipitate was resuspended with a marker diluent in a volume which is equal to that of the supernatant solution to prepare the COVID-19 fluorescence marker.

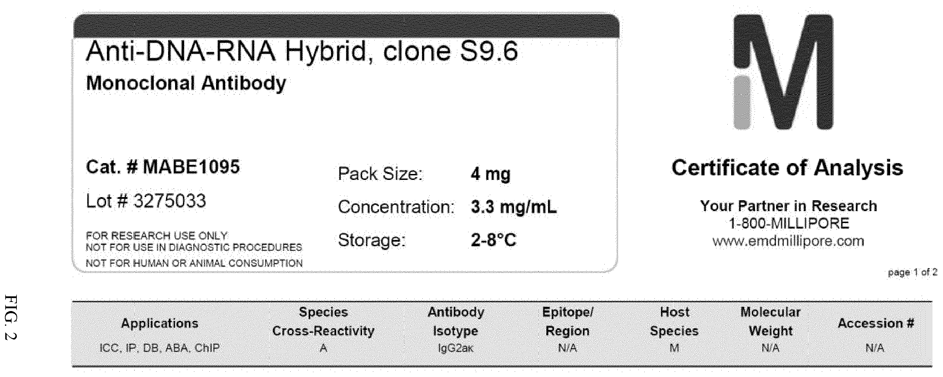

The used fluorescence signal is from a fluorescent microsphere. The protein is S9.6 protein produced by Merck, and the specific model of the protein is referenced to FIG. 2.

(3) After the detection sample entered the reaction tube, the reaction liquid powder was redissolved under the action of the preserving liquid in the detection sample to form a liquid phase environment. When the redissolving liquid of the reaction liquid was insufficient, the redissolving liquid may be properly added, wherein the adding amount is 85µL±5%, and the redissolving liquid is generally normal saline or normal saline added with a bactericidal component. After the materials were mixed in the reaction tube, the reaction tube was placed in a heater and heated for 37±2°C. In this liquid phase environment, two reactions were carried out, namely:
a. DNA recognized, hybridized, captured and combined a target RNA fragment into a DNA-RNA complex; and b. protein 1 specifically recognized and combined the DNA-RNA complex to form a DNA-RNA-protein-fluorescence signal complex which is defined as a reaction complex.

(4) The reaction complex was taken out and added into a detection reagent card, and was chromatographed on a test strip under the action of auxiliary liquid until a T line, wherein the T line is coated with S9.6 protein produced by Merck. When the COVID-19 virus was detected in the sample, there would be the DNA-RNA-protein-fluorescence signal complex in the reaction complex. The S9.6 protein produced by Merck may specifically recognize and combine the DNA-RNA complex, so the DNA-RNA-protein-fluorescence signal complex in the reaction complex may be fixed on the T line to form a "protein-DNA-RNA-protein-fluorescence signal", a fluorescence band was formed on the T line, and the fluorescence signal may be detected on the T line through an instrument. The reaction complex passed through the T line and then was continuously chromatographed to pass through a C line, the C line was coated with a rabbit polyclonal antibody, and the rabbit polyclonal antibody may non-specifically combine the S9.6 protein produced by Merck, so on the premise of excessive amount, a certain amount of protein connected with the fluorescence signal would be captured theoretically to form a fluorescence band, which may be recognized through an instrument. The above-mentioned T line marker and C line marker are precisely S9.6 protein produced by Merck and coated on the T line and a rabbit polyclonal antibody coated on the C line, and all the protein are not marked with the fluorescence signals.

The specific experimental data contents of the invention are:

First part experiment preparation

The main research methods of each performance are all combined with the evaluation method actually adopted in China, including positive reference product coincidence rate and negative reference product coincidence rate of products, minimum detection limit, precision, interference experiment, cross reaction and the like, to complete experimental test.

**Table 3 Material information required for experiment**

| Serial number | Name | Batch number/model number | Status |
|---|---|---|---|
| 1 | 2019 novel coronavirus (2019-nCoV) nucleic acid assay kit (hybrid capture immunofluorescence method) | / | |
| 2 | Immunoassay analyzer | | |
| 3 | 2019-nCoV reference product | | / |

The information of samples and other materials used in the analytical performance evaluation test is shown in the following table:

**Table 4 The information of samples and other materials used in the analytical performance evaluation test**

| Serial number | Name |
|---|---|
| 1 | 2019-nCoV Throat swab and sputum samples (positive, negative and critically positive |
| | samples) |
| 2 | Interferent -endogenous |
| 3 | Interferent-medicine |
| 4 | Cross-pathogenic microorganism |
| 5 | Cross sample (Samples collected from different regions with positive endemic coronavirus or positive common respiratory pathogen and negative COVID-19) |

Second part Positive/negative reference product coincidence rate

### 1. Standard requirement

### 1.1 Positive reference product coincidence rate

13 positive reference products (P1-P13) were detected, including 5 positive samples in the 2019-CoV N segment, the 2019-CoV E segment and the 2019-CoV ORF lab segment and 5 positive samples in sputum and throat swab liquid, and the results should be positive.

### 1.2 Negative reference product coincidence rate

19 negative reference products (N1-N19) were detected, and the results should be negative.

**Table 5 Information of 19 negative reference products (N1-N19)**

| Serial Number | Reference product Information | Type |
|---|---|---|
| N5 | Negative for 2019-nCoV and positive for influenza A virus | Throat swab liquid |
| N6 | Negative for 2019-nCoV and positive for metapneumovirus | Throat swab liquid |
| N7 | Negative for 2019-nCoV and positive for influenza B virus | Throat swab liquid |
| N8 | Negative for 2019-nCoV and positive for parainfluenza virus | Throat swab liquid |
| N9 | Negative for 2019-nCoV and positive for respiratory syncytial virus | Throat swab liquid |
| N10 | Negative for 2019-nCoV and positive for rhinovirus | Throat swab liquid |
| N11 | Negative for 2019-nCoV and positive for adenovirus | Throat swab liquid |
| N12 | Negative for 2019-nCoV and positive for mycoplasma pneumoniae | Throat swab liquid |
| N13 | Negative for 2019-nCoV and positive for chlamydia pneumoniae | Throat swab liquid |
| N14 | Negative for 2019-nCoV Positive for human coronavirus 229E N segment | Throat swab liquid |
| N15 | Negative for 2019-nCoV Positive for human coronavirus NL63 N segment | Throat swab liquid |
| N16 | Negative for 2019-nCoV Positive for human coronavirus OC43 N segment | Throat swab liquid |
| N17 | Negative for 2019-nCoV Positive for human coronavirus HKU1 N segment | Throat swab liquid |
| N18 | Negative for 2019-nCoV | Pseudovirus |
| | Positive for human coronavirus MERS N segment | |
| N19 | Negative for 2019-nCoV Positive for human coronavirus SARS N segment | Pseudovirus |

### 2. Method

8 positive reference products (P1-P8) and 19 negative reference products (N1-N19) were detected for one time by the 2019 novel coronavirus (2019-nCoV) nucleic acid assay kit (hybrid capture immunofluorescence method).

### 3. Result

**Table 6 Results of reagent positive/negative reference product coincidence rate**

| Batch number | 401200101 | |
|---|---|---|
| P1 | 196.77 | + |
| P2 | 178.78 | + |
| P3 | 173.80 | + |
| P4 | 185.63 | + |
| P5 | 132.54 | + |
| P6 | 451.26 | + |
| P7 | 232.15 | + |
| P8 | 110.23 | + |
| P9 | 105.46 | + |
| P10 | 129.75 | + |
| P11 | 206.18 | + |
| P12 | 251.46 | + |
| P13 | 512.48 | + |
| N1 | 45.35 | - |
| N2 | 21.40 | - |
| N3 | 31.98 | - |
| N4 | 55.50 | - |
| N5 | 55.12 | - |
| N6 | 26.08 | - |
| N7 | 57.01 | - |
| N8 | 49.51 | - |
| N9 | 55.62 | - |
| N10 | 26.87 | - |
| N11 | 51.33 | - |
| N12 | 20.59 | - |
| N13 | 41.55 | - |
| N14 | 52.12 | - |
| N15 | 39.56 | - |
| N16 | 60.48 | - |
| N17 | 68.45 | - |
| N18 | 32.15 | - |
| N19 | 29.52 | - |

### 4. Conclusion

The detection results of 13 positive reference products and 19 negative reference products of three batches of reagents all meet the proposed standard.

### Third part Determination of minimum detection limit

### 1. Objective

The minimum detection limit of the 2019 novel coronavirus (2019-nCoV) nucleic acid assay kit (hybrid capture immunofluorescence method) is determined.

### 2. Material information

**Table 7 Material information required for experiment for determining the minimum detection limit**

| Serial number | Name | Use |
|---|---|---|
| 1 | Pseudovirus (including the 2019-CoV N segment, the 2019-CoV E segment and the 2019-CoV ORF lab segment) | Establishment of the minimum detection limit |
| 2 | 2019-nCoV Throat swab and sputum positive samples | Establishment of the minimum detection limit Verification of the minimum detection limit |
| 3 | 2019-nCoV Throat swab and sputum positive samples | Containment verification of the minimum detection limit |

### 3. Establishment

### 3.1 Preparation of pseudovirus sample

The pseudoviruses at 3 segments (including the 2019-CoV N segment, the 2019-CoV E segment and the 2019-CoV ORF lab segment) were gradiently diluted with mixed negative samples as diluent respectively into 5000 copies/mL, 2500 copies/m, 1000 copies/mL,800 copies/mL, 500 copies/mL, 250 copies/mL and 100 copies/mL.

### 3.3 Sample measurement

The samples (pseudovirus, throat swab sample and sputum sample) were measured by the 2019 novel coronavirus (2019-nCoV) nucleic acid assay kit (hybrid capture immunofluorescence method), each sample was tested for 20 times, and the positive detection rate was detected.

### 3.4. Determination

The minimum concentration with positive detection rate more than or equal to 95% is the minimum detection limit of this segment.

### 3.5 Sample detection

**Table 8 Measurement result of each dilution sample**

| | **5000** | | **2500** | | **1000** | | **800** | | **500** | | **250** | | **100** | |
|---|---|---|---|---|---|---|---|---|---|---|---|---|---|---|
| 1 | 478.70 | + | 140.58 | + | 102.13 | + | 86.19 | - | 101.27 | + | 81.04 | - | 65.58 | - |
| 2 | 470.76 | + | 145.56 | + | 104.30 | + | 104.31 | + | 99.59 | - | 84.15 | - | 76.33 | - |
| 3 | 489.00 | + | 145.04 | + | 101.65 | + | 96.94 | - | 100.31 | + | 107.11 | + | 64.87 | - |
| 4 | 487.85 | + | 153.84 | + | 100.36 | + | 109.97 | + | 83.41 | - | 85.45 | - | 59.10 | - |
| 5 | 455.52 | + | 147.96 | + | 101.73 | + | 95.16 | - | 106.86 | + | 89.87 | - | 55.45 | - |
| 6 | 479.44 | + | 152.99 | + | 109.75 | + | 101.23 | + | 104.12 | + | 81.07 | - | 55.88 | - |
| 7 | 480.61 | + | 153.19 | + | 106.66 | + | 105.74 | + | 93.48 | - | 75.35 | - | 71.57 | - |
| 8 | 487.43 | + | 157.88 | + | 100.94 | + | 88.88 | - | 89.42 | - | 74.81 | - | 59.89 | - |
| 9 | 498.15 | + | 159.06 | + | 104.81 | + | 105.03 | + | 94.98 | - | 105.26 | + | 102.34 | + |
| 10 | 474.14 | + | 143.43 | + | 104.61 | + | 107.44 | + | 99.14 | - | 109.91 | + | 66.23 | - |
| 11 | 482.48 | + | 141.57 | + | 104.50 | + | 108.94 | + | 108.89 | + | 84.92 | - | 62.85 | - |
| 12 | 482.56 | + | 146.63 | + | 105.71 | + | 96.15 | - | 97.19 | - | 88.88 | - | 58.56 | - |
| 13 | 486.82 | + | 150.40 | + | 108.79 | + | 98.33 | - | 105.89 | + | 80.79 | - | 58.68 | - |
| 14 | 477.20 | + | 142.32 | + | 106.22 | + | 107.61 | + | 83.54 | - | 107.36 | + | 74.67 | - |
| 15 | 495.41 | + | 144.33 | + | 102.70 | + | 107.33 | + | 88.86 | - | 88.28 | - | 64.59 | - |
| 16 | 490.73 | + | 154.63 | + | 109.02 | + | 106.97 | + | 103.16 | + | 79.22 | - | 55.57 | - |
| 17 | 497.68 | + | 154.73 | + | 107.92 | + | 107.89 | + | 105.65 | + | 80.52 | - | 65.79 | - |
| 18 | 482.99 | + | 155.77 | + | 103.52 | + | 106.34 | + | 92.23 | - | 106.15 | - | 63.13 | - |
| 19 | 455.93 | + | 153.44 | + | 107.27 | + | 102.48 | + | 102.37 | + | 89.60 | - | 50.94 | - |
| 20 | 470.77 | + | 144.19 | + | 107.99 | + | 108.58 | + | 103.45 | + | 72.95 | - | 64.80 | - |
| Positive rate | **100%** | | **100%** | | **100%** | | **70%** | | **50%** | | **20%** | | **5%** | |

The above result shows that the positive detection rate of the samples with the minimum detection limit concentration of 1000copies/mL is more than or equal to 95%.

### 6. Conclusion

In conclusion, when the concentration of each sample of the three batches of reagents is 1000 copies/mL, the positive detection rate is more than or equal to 95%, so the minimum detection limit of the product is 1000 copies/mL.

### Fourth part Precision test

### 1. Standard requirement

(1) The intra-batch precision variable coefficient CV: ≤ 10%, and the results are consistent.
(2) The inter-batch precision variable coefficient CV: ≤ 10%, and the results are consistent.
(3) The intermediate precision variable coefficient CV:≤ 10%, and the results are consistent.

### 2. Material information

**Table 9 Material information required for experiment for determining precision**

| Serial number | Name |
|---|---|
| 1 | Pseudovirus including the 2019-CoV N segment, the 2019-CoV E segment and the |
| | 2019-CoV ORF lab segment |
| 2 | 2019-nCoV Throat swab and sputum samples |

### 3. Method

### 3.1 Precision reference product measurement

Three batches of 2019 novel coronavirus (2019-nCoV) nucleic acid assay kits (hybrid capture immunofluorescence method) were detected by three precision reference products respectively.

### 20 groups of data were acquired for precision analysis.

### 4. Result

### 4.1 Precision reference product result

**Table 10 Three batches of reagent verification precision reference product J1 measurement result**

| | | | | | | | |
|---|---|---|---|---|---|---|---|
| First day | 1-1-1 | 143.19 | + | 156.02 | + | 131.95 | + |
| | 1-1-2 | 143.01 | + | 153.39 | + | 137.42 | + |
| | 1-2-1 | 134.16 | + | 136.72 | + | 131.79 | + |
| | 1-2-2 | 150.56 | + | 134.26 | + | 130.85 | + |
| | 2-1-1 | 145.93 | + | 149.41 | + | 140.14 | + |
| | 2-1-2 | 152.03 | + | 140.68 | + | 138.75 | + |
| | 2-2-1 | 158.56 | + | 141.92 | + | 158.16 | + |
| | 2-2-2 | 131.32 | + | 157.13 | + | 132.32 | + |
| | 3-1-1 | 135.36 | + | 141.42 | + | 138.86 | + |
| | 3-1-2 | 136.08 | + | 132.32 | + | 159.34 | + |
| | 3-2-1 | 141.42 | + | 157.72 | + | 158.81 | + |
| | 3-2-2 | 134.51 | + | 141.70 | + | 135.92 | + |
| | 4-1-1 | 136.09 | + | 144.78 | + | 156.89 | + |
| | 4-1-2 | 145.27 | + | 142.62 | + | 154.15 | + |
| | 4-2-1 | 155.52 | + | 142.85 | + | 151.29 | + |
| | 4-2-2 | 153.74 | + | 139.87 | + | 146.83 | + |
| Second day | 1-1-1 | 148.31 | + | 137.72 | + | 157.34 | + |
| | 1-1-2 | 151.80 | + | 154.87 | + | 150.28 | + |
| | 1-2-1 | 138.01 | + | 130.39 | + | 148.42 | + |
| | 1-2-2 | 155.91 | + | 154.96 | + | 149.98 | + |
| | 2-1-1 | 152.78 | + | 157.30 | + | 144.49 | + |
| | 2-1-2 | 145.49 | + | 158.40 | + | 140.56 | + |
| | 2-2-1 | 146.53 | + | 137.79 | + | 135.37 | + |
| | 2-2-2 | 130.37 | + | 154.64 | + | 141.50 | + |
| | 3-1-1 | 145.45 | + | 140.39 | + | 133.83 | + |
| | 3-1-2 | 143.19 | + | 130.66 | + | 143.13 | + |
| | 3-2-1 | 143.66 | + | 142.10 | + | 158.38 | + |
| | 3-2-2 | 158.37 | + | 136.07 | + | 143.23 | + |
| | 4-1-1 | 151.99 | + | 147.89 | + | 145.84 | + |
| | 4-1-2 | 141.77 | + | 141.29 | + | 157.12 | + |
| | 4-2-1 | 150.47 | + | 140.28 | + | 149.00 | + |
| | 4-2-2 | 159.64 | + | 135.15 | + | 145.85 | + |
| Third day | 1-1-1 | 139.12 | + | 142.97 | + | 142.22 | + |
| | 1-1-2 | 133.25 | + | 141.35 | + | 158.86 | + |
| | 1-2-1 | 151.58 | + | 146.31 | + | 152.48 | + |
| | 1-2-2 | 154.63 | + | 149.21 | + | 130.30 | + |
| | 2-1-1 | 140.32 | + | 134.87 | + | 154.00 | + |
| | 2-1-2 | 144.45 | + | 154.14 | + | 135.14 | + |
| | 2-2-1 | 135.08 | + | 137.08 | + | 142.94 | + |
| | 2-2-2 | 136.90 | + | 131.04 | + | 134.50 | + |
| | 3-1-1 | 144.70 | + | 147.85 | + | 154.06 | + |
| | 3-1-2 | 139.18 | + | 159.91 | + | 134.30 | + |
| | 3-2-1 | 141.10 | + | 133.03 | + | 133.34 | + |
| | 3-2-2 | 146.53 | + | 146.77 | + | 145.76 | + |
| | 4-1-1 | 140.10 | + | 137.69 | + | 141.26 | + |
| | 4-1-2 | 142.40 | + | 130.33 | + | 156.42 | + |
| | 4-2-1 | 140.03 | + | 150.03 | + | 146.48 | + |
| | 4-2-2 | 130.83 | + | 134.23 | + | 149.10 | + |
| Fourth day | 1-1-1 | 152.65 | + | 151.81 | + | 155.90 | + |
| | 1-1-2 | 140.07 | + | 135.34 | + | 146.27 | + |
| | 1-2-1 | 150.05 | + | 152.27 | + | 131.48 | + |
| | 1-2-2 | 144.68 | + | 146.04 | + | 141.58 | + |
| | 2-1-1 | 130.93 | + | 138.65 | + | 147.41 | + |
| | 2-1-2 | 134.82 | + | 151.13 | + | 147.92 | + |
| | 2-2-1 | 157.65 | + | 155.10 | + | 138.68 | + |
| | 2-2-2 | 146.24 | + | 155.91 | + | 143.73 | + |
| | 3-1-1 | 130.95 | + | 132.50 | + | 152.39 | + |
| | 3-1-2 | 159.01 | + | 143.13 | + | 149.69 | + |
| | 3-2-1 | 158.36 | + | 156.77 | + | 134.08 | + |
| | 3-2-2 | 155.97 | + | 133.15 | + | 157.96 | + |
| | 4-1-1 | 141.81 | + | 155.90 | + | 137.40 | + |
| | 4-1-2 | 135.39 | + | 140.10 | + | 131.87 | + |
| | 4-2-1 | 139.12 | + | 131.49 | + | 131.19 | + |
| | 4-2-2 | 149.88 | + | 134.67 | + | 136.08 | + |
| Fifth day | 1-1-1 | 139.58 | + | 144.48 | + | 132.37 | + |
| | 1-1-2 | 145.98 | + | 141.89 | + | 144.37 | + |
| | 1-2-1 | 140.56 | + | 142.87 | + | 150.13 | + |
| | 1-2-2 | 146.3 1 | + | 142.76 | + | 145.09 | + |
| | 2-1-1 | 155.69 | + | 136.51 | + | 132.72 | + |
| | 2-1-2 | 146.21 | + | 157.54 | + | 150.66 | + |
| | 2-2-1 | 142.93 | + | 136.56 | + | 140.59 | + |
| | 2-2-2 | 152.48 | + | 150.50 | + | 157.51 | + |
| | 3-1-1 | 143.65 | + | 133.76 | + | 135.82 | + |
| | 3-1-2 | 133.17 | + | 143.60 | + | 133.49 | + |
| | 3-2-1 | 134.58 | + | 141.09 | + | 146.31 | + |
| | 3-2-2 | 140.71 | + | 156.01 | + | 142.23 | + |
| | 4-1-1 | 136.64 | + | 148.33 | + | 156.66 | + |
| | 4-1-2 | 156.38 | + | 138.11 | + | 142.04 | + |
| | 4-2-1 | 141.65 | + | 148.78 | + | 155.72 | + |
| | 4-2-2 | 146.64 | + | 156.64 | + | 150.29 | + |
| Average value^{X} | | 144.39 | | 144.04 | | 144.48 | |
| Standard deviation (SD) | | 7.98 | | 8.52 | | 8.81 | |
| Intra-batch CV | | 5.53% | | 5.91% | | 6.10% | |
| Inter-batch CV | | 5.83% | | | | | |

### 4.2 5. Conclusion

The products were respectively measured for precision reference products, the variable coefficient CV is not greater than 10%, and the inter-batch variable coefficient CV is not greater than 10%.

### Fifth part Endogenous interference experiment

### 1. Objective

The influence on the detection result of the 2019 novel coronavirus (2019-nCoV) nucleic acid assay kit (hybrid capture immunofluorescence method) by endogenous interference substances such as hemoglobin and mucoprotein in the common samples was analyzed, and the anti-interference performance of the products was evaluated.

### 2. Material information

**Table 11 Material information required for endogenous interference experiment**

| Serial number | Name |
|---|---|
| 1 | 2019-nCoV Throat swab |
| 2 | Hemoglobin |
| 3 | Mucoprotein |

### 3. Method

### 3.1 Selection of throat swab

### 3.2 Preparation of basic sample

### Basic sample: clinical sample 0.072mL+0.008mL normal saline

### 3.2 Preparation of Interference sample

The following interferents were added into the above samples respectively to prepare the corresponding interference samples:
Hemoglobin interference sample: Sample 0.072mL+0.008mL 20g/L hemoglobin
Sample 0.072mL+0.008mL 10g/L hemoglobin
Sample 0.072mL+0.008mL 5g/L hemoglobin
Mucoprotein interference sample: Sample 0.072mL+0.008mL 200mg/mL mucoprotein
Sample 0.072mL+0.008mL 100mg/mL mucoprotein
Sample 0.072mL+0.008mL 50mg/mL mucoprotein

### 3.3 Measurement

The above preparation samples were detected by the 2019 novel coronavirus (2019-nCoV) nucleic acid assay kits (hybrid capture immunofluorescence method) respectively, and a ratio of the interference sample detection value to the basic sample detection value was calculated.

### 4. Result

### 4.1 Throat swab sample interference result

### 4.1.2 Hemoglobin interference detection result

**Table 12 Three batches of reagent throat swab negative sample interference experiment (interferent: hemoglobin) detection result**

| Critic ally positi | Interfer ent concen | 401200101 | | | 401200102 | | | 401200103 | | |
|---|---|---|---|---|---|---|---|---|---|---|
| | | Basic value | Interf erence | Rati o | Basic value | Interfe rence | Rati o | Basic value | Interfe rence | Rati o |
| L1 | 2g/L | 125.48 | 130.5 | 1.04 | 126.73 | 128.00 | 1.01 | 129.24 | 132.17 | 1.02 |
| | 1g/L | 110.37 | 114.7 | 1.04 | 109.27 | 108.17 | 0.99 | 101.54 | 105.46 | 1.04 |
| | 0.5g/L | 100.45 | 102.4 | 1.02 | 103.46 | 107.88 | 1.04 | 108.49 | 112.83 | 1.04 |
| L2 | 2g/L | 110.64 | 102.9 | 0.93 | 109.53 | 120.49 | 1.10 | 120.60 | 129.04 | 1.07 |
| | 1g/L | 121.80 | 109.6 | 0.90 | 125.45 | 117.93 | 0.94 | 125.45 | 124.20 | 0.99 |
| | 0.5g/L | 127.29 | 115.8 | 0.91 | 133.65 | 124.30 | 0.93 | 124.74 | 116.01 | 0.93 |
| L3 | 2g/L | 100.72 | 106.6 | 1.06 | 104.52 | 106.88 | 1.02 | 108.78 | 110.95 | 1.02 |
| | 1g/L | 100.65 | 108.6 | 1.08 | 102.66 | 103.69 | 1.01 | 116.58 | 119.53 | 1.03 |
| | 0.5g/L | 125.66 | 119.3 | 0.95 | 130.69 | 142.45 | 1.09 | 116.86 | 114.53 | 0.98 |

### 5. Conclusion

The interference samples prepared by the above samples and the corresponding basic samples have the consistent detection result, which shows that the hemoglobin with the concentration content of 2g/L and the mucoprotein with concentration content of 20mg/mL in the samples do not affect the detection.

### Sixth part Cross reaction experiment

### 1. Objective

The influence on the detection result of the 2019 novel coronavirus (2019-nCoV) nucleic acid assay kit (hybrid capture immunofluorescence method) by the common respiratory pathogen was analyzed, and the specificity of the products was evaluated.

### 2. Material information

**Table 13 Material information required for cross reaction experiment**

| 1 | 2019-nCoV Throat swab sample |
|---|---|
| 2 | 2019-nCoV |
| 3 | Various pathogenic microorganisms |
| 4 | Cross samples (samples collected from different regions with positive endemic coronavirus or positive common respiratory pathogen and negative COVID-19) |

### 3. Method

### 3.1.2 Preparation of basic sample

The basic sample is directly applied to detection and the detection result is a basic value.

### Basic sample: basic sample 0.09mL+0.01mL normal saline

Preparation of the sample, that is, preparation of 2 cases of negative samples and 2 cases of critically positive samples crossed by each pathogenic microorganism.

### 4) Preparation method of cross sample

### Virus cross sample: sample 0.072mL+0.008mL 106pfu/mL

Cross samples of bacteria, mycoplasma and chlamydia: sample 0.072mL+0.008mL 107cfu/mL

**Table 14 Various pathogenic microorganism information**

| Serial Number | Pathogen | Species | Concentration | Titer test | Cross concentration |
|---|---|---|---|---|---|
| 1 | H1N1 (Novel influenza A H1N1 virus (2009)) | A-H1N1-2009 | 10⁶pfu/mL | Plaque test | 10⁵pfu/m L |
| 2 | Seasonal H1N1 influenza virus | A-H1N1 | 10⁶pfu/mL | Plaque test | 10⁵pfu/m L |
| 3 | H3N2 | A-H3N2 | 10⁶pfu/mL | Plaque test | 10⁵pfu/m L |
| 4 | H5N1 | A-H5N1 | 10⁶pfu/mL | Plaque test | 10⁵pfu/m L |
| 5 | H7N9 | A-H7N9 | 10⁶pfu/mL | Plaque test | 10⁵pfu/m L |
| 6 | Influenza B virus Yamagata | B-Yamagata | 10⁶pfu/mL | Plaque test | 10⁵pfu/m L |
| 7 | Influenza B virus Victoria | B-Victoria | 10⁶pfu/mL | Plaque test | 10⁵pfu/m L |
| 8 | Respiratory syncytial virus type A | RSV-A2 | 10⁶pfu/mL | Plaque test | 10⁵pfu/m L |
| 9 | Respiratory syncytial virus type B | RSV-B | 10⁶pfu/mL | Plaque test | 10⁵pfu/m L |
| 10 | Enterovirus A | CV-A10 | 10⁶pfu/mL | Plaque test | 10⁵pfu/m L |
| 11 | Enterovirus B | Echovirus 6 | 10⁶pfu/mL | Plaque test | 10⁵pfu/m L |
| 12 | Enterovirus C | CV-A21 | 10⁶pfu/mL | Plaque test | 10⁵pfu/m L |
| 13 | Enterovirus D | EV-D68 | 10⁶pfu/mL | Plaque test | 10⁵pfu/m L |
| 14 | Parainfluenza virus type 1 | HPIVs-1 | 10⁶pfu/mL | Plaque test | 10⁵pfu/m L |
| 15 | Parainfluenza virus type 2 | HPIVs-2 | 10⁶pfu/mL | Plaque test | 10⁵pfu/m L |
| 16 | Parainfluenza virus type 3 | HPIVs-3 VR-93 | 10⁶pfu/mL | Plaque test | 10⁵pfu/m L |
| 17 | Rhinovirus A | HRV-9 VR-489 | 10⁶pfu/mL | Plaque test | 10⁵pfu/m L |
| 18 | Rhinovirus B | HRV-52 VR-1162 HRV-3 VR-1113 | 10⁶pfu/mL | Plaque test | 10⁵pfu/m L |
| 19 | Rhinovirus C | HRV-16 VR-283 | 10⁶pfu/mL | Plaque test | 10⁵pfu/m L |
| 20 | Adenovirus type 1 | HAdV-1 VR-1 | 10⁶pfu/mL | Plaque test | 10⁵pfu/m L |
| 21 | Adenovirus type 2 | HAdV-2 VR-846 | 10⁶pfu/mL | Plaque test | 10⁵pfu/m L |
| 22 | Adenovirus type 3 | HAdV-3 | 10⁶pfu/mL | Plaque test | 10⁵pfu/m L |
| 23 | Adenovirus type 4 | HAdV-4 VR-1572 | 10⁶pfu/mL | Plaque test | 10⁵pfu/m L |
| 24 | Adenovirus type 5 | HAdV-5 VR-1578/151 6 | 10⁶pfu/mL | Plaque test | 10⁵pfu/m L |
| 25 | Adenovirus type 7 | HAdV-7 VR-7 | 10⁶pfu/mL | Plaque test | 10⁵pfu/m L |
| 26 | Adenovirus type 55 | HAdV-55 | 10⁶pfu/mL | Plaque test | 10⁵pfu/m L |
| 27 | Human interstitial pneumonia | HMPV | 10⁶pfu/mL | Plaque test | 10⁵pfu/m L |
| 28 | Human metapneumovirus | HMPV | 10⁶pfu/mL | Plaque test | 10⁵pfu/m L |
| 29 | EB virus | HHV-4 VR-1492 | 10⁶pfu/mL | Plaque test | 10⁵pfu/m L |
| 30 | Measles virus | MV VR-24 | 10⁶pfu/mL | Plaque test | 10⁵pfu/m L |
| 31 | Human cytomegalovirus | HHV-5 VR-977 | 10⁶pfu/mL | Plaque test | 10⁵pfu/m L |
| 32 | Rotavirus | RV VR-2018 | 10⁶pfu/mL | Plaque test | 10⁵pfu/m L |
| 33 | Norovirus | NOR | 10⁶pfu/mL | Plaque test | 10⁵pfu/m L |
| 34 | Mumps virus | MuV VR-106 | 10⁶pfu/mL | Plaque test | 10⁵pfu/m L |
| 35 | Varicella-zoster virus | VZV VR-1367 | 10⁶pfu/mL | Plaque test | 10⁵pfu/m L |
| 36 | Legionella | 33152 | 10⁷cfu/mL | Colony counting | 10⁶cfu/m L |
| 37 | Bordetella pertussis | BAA-589 | 10⁷cfu/mL | Colony counting | 10⁶cfu/m L |
| 38 | Haemophilus influenzae | Hib | 10⁷cfu/mL | Colony counting | 10⁶cfu/m L |
| 39 | Staphylococcus aureus | CGMCC 1.2910 | 10⁷cfu/mL | Colony counting | 10⁶cfu/m L |
| 40 | Sreptococcus pneumoniae | CGMCC 1.8722 | 10⁷cfu/mL | Colony counting | 10⁶cfu/m L |
| 41 | Pyogenic streptococcus | CGMCC 1.8868 | 10⁷cfu/mL | Colony counting | 10⁶cfu/m L |
| 42 | Klebsiella pneumoniae | CGMCC 1.1736 | 10⁷cfu/mL | Colony counting | 10⁶cfu/m L |
| 43 | Mycobacterium tuberculosis | 25177 | 10⁷cfu/mL | Colony counting | 10⁶cfu/m L |
| 44 | Mycoplasma pneumoniae | 39505 | 10⁷cfu/mL | Colony counting | 10⁶cfu/m L |
| 45 | Chlamydia pneumoniae | VR-2282 | 10⁷cfu/mL | Colony counting | 10⁶cfu/m L |
| 46 | Aspergillus fumigatus | AF293 | 10⁷cfu/mL | Colony counting | 10⁶cfu/m L |
| 47 | Candida albicans | SC5314 | 10⁷cfu/mL | Colony counting | 10⁶cfu/m L |
| 48 | Candida glabrata | ATCC 2001 | 10⁷cfu/mL | Colony counting | 10⁶cfu/m L |
| 49 | Cryptococcus neoformans | H99 | 10⁷cfu/mL | Colony counting | 10⁶cfu/m L |
| 50 | Cryptococcus Gattii | R265 | 10⁷cfu/mL | Colony counting | 10⁶cfu/m L |
| 51 | Coronavirus 229E | VR-740 | 10⁶pfu/mL | Plaque test | 10⁵pfu/m L |
| 52 | Coronavirus OC43 | VR-1558 | 10⁶pfu/mL | Plaque test | 10⁵pfu/m L |
| 53 | Coronavirus NL63 | COV-NL63 | 10⁶pfu/mL | Plaque test | 10⁵pfu/m L |
| 54 | Coronavirus HKU1 | COV-HKU1 | 10⁶pfu/mL | Plaque test | 10⁵pfu/m L |
| 55 | Coronavirus MERS | MERS | 10⁸TU/mL | Digital PCR | 10⁷TU/m L |
| 56 | Coronavirus SARS | SARS | 10⁸TU/mL | Digital PCR | 10⁷TU/m L |

### 3.1.4 Measurement

The above preparation samples were detected by the 2019 novel coronavirus (2019-nCoV) nucleic acid assay kits (hybrid capture immunofluorescence method) respectively, and a ratio of the cross sample detection value to the basic sample detection value was calculated.

**Table 15 Reagent cross experiment detection result**

| Pathogenic microorganism | | | |
|---|---|---|---|
| | Basic value | Interference value | Ratio |
| H1N1 (Novel influenza A H1N1 virus (2009)) | 52.81 | 53.45 | 1.01 |
| Seasonal H1N1 influenza virus | 54.61 | 50.41 | 0.92 |
| H3N2 | 54.24 | 52.31 | 0.96 |
| H5N1 | 45.59 | 42.05 | 0.92 |
| H7N9 | 42.99 | 43.74 | 1.02 |
| Influenza B virus Yamagata | 47.67 | 46.99 | 0.99 |
| Influenza B virus Victoria | 49.92 | 50.76 | 1.02 |
| Respiratory syncytial virus type A | 43.69 | 45.32 | 1.04 |
| Respiratory syncytial virus type B | 49.17 | 44.86 | 0.91 |
| Enterovirus A | 57.68 | 53.47 | 0.93 |
| Enterovirus B | 46.6 | 45.68 | 0.98 |
| Enterovirus C | 50.71 | 49.39 | 0.97 |
| Enterovirus D | 57.93 | 54.99 | 0.95 |
| Parainfluenza virus type 1 | 42.49 | 39.15 | 0.92 |
| Parainfluenza virus type 2 | 53.43 | 52.17 | 0.98 |
| Parainfluenza virus type 3 | 57.72 | 62.95 | 1.09 |
| Rhinovirus A | 49.79 | 51.42 | 1.03 |
| Rhinovirus B | 43.52 | 46.82 | 1.08 |
| Rhinovirus C | 56.86 | 61.42 | 1.08 |
| Adenovirus type 1 | 55.88 | 53.51 | 0.96 |
| Adenovirus type 2 | 44.13 | 43.11 | 0.98 |
| Adenovirus type 3 | 41.84 | 39.89 | 0.95 |
| Adenovirus type 4 | 47.54 | 51.11 | 1.08 |
| Adenovirus type 5 | 53.79 | 55.41 | 1.03 |
| Adenovirus type 7 | 47.45 | 52.05 | 1.10 |
| Adenovirus type 55 | 51.2 | 48.58 | 0.95 |
| Human interstitial pneumonia | 46.47 | 41.84 | 0.90 |
| human metapneumovirus | 54.15 | 57.07 | 1.05 |
| EB virus | 46.21 | 43.99 | 0.95 |
| Measles virus | 40.82 | 41.17 | 1.01 |
| Human cytomegalovirus | 50.31 | 46.43 | 0.92 |
| Rotavirus | 54.68 | 53.76 | 0.98 |
| Norovirus | 44.1 | 47.86 | 1.09 |
| Mumps virus | 51.54 | 51.25 | 0.99 |
| Varicella-zoster virus | 59.03 | 62.39 | 1.06 |
| Legionella | 47.01 | 45.7 | 0.97 |
| Bordetella pertussis | 43.73 | 40.61 | 0.93 |
| Haemophilus influenzae | 43.67 | 40.46 | 0.93 |
| Staphylococcus aureus | 54.98 | 58.07 | 1.06 |
| Streptococcus pneumoniae | 57.51 | 58.41 | 1.02 |
| Pyogenic streptococcus | 49.55 | 52.47 | 1.06 |
| Klebsiella pneumoniae | 51.69 | 55.87 | 1.08 |
| Mycobacterium tuberculosis | 47.07 | 47.6 | 1.01 |
| Mycoplasma pneumoniae | 47.12 | 51.82 | 1.10 |
| Chlamydia pneumoniae | 53.49 | 54.68 | 1.02 |
| Aspergillus fumigatus | 58.82 | 62.28 | 1.06 |
| Candida albicans | 58.43 | 58.14 | 1.00 |
| Candida glabrata | 40.27 | 37.9 | 0.94 |
| Cryptococcus neoformans | 45.39 | 45.35 | 1.00 |
| Cryptococcus Gattii | 43.04 | 42.55 | 0.99 |
| Coronavirus 229E | 45.69 | 41.78 | 0.91 |
| Coronavirus OC43 | 47.4 | 51.15 | 1.08 |
| Coronavirus NL63 | 49.16 | 48.21 | 0.98 |
| Coronavirus HKU1 | 54.97 | 58.85 | 1.07 |
| Coronavirus MERS | 40.75 | 37.32 | 0.92 |
| Coronavirus SARS | 44.39 | 40.27 | 0.91 |

### 5. Conclusion

In conclusion, the detection results of the cross sample prepared by the above sample and the corresponding basic sample are consistent, a ratio of the cross sample detection value to the basic sample detection value is between 0.9 and 1.1, the common pathogenic microorganisms in different regions are positive, and the detection result of the 2019-nCoV negative samples are negative, indicating that there is no cross phenomenon between the reagent and the following common respiratory pathogens.

**Table 16 The common respiratory pathogens which do not cross with the 2019-nCoV negative sample**

| H1N1 (Novel influenza A H1N1 virus (2009)) | Seasonal H1N1 influenza virus | H3N2 influenza virus | H5N1 avian influenza virus | H7N9 avian influenza virus | Influenza B virus Yamagata | Influenza B virus Victoria |
|---|---|---|---|---|---|---|
| Respiratory syncytial virus type A | Respiratory syncytial virus type B | Enteroviru sA | Enterovirus B | Enteroviru sC | Enterovirus D | Parainflue nza virus type 1 |
| Parainfluen za virus type 2 | Parainfluen za virus type 3 | Rhinoviru sA | Rhinovirus B | Rhinoviru sC | Adenovirus type 1 | Adenoviru s type 2 |
| Adenovirus type 3 | Adenovirus type 4 | Adenoviru s type 5 | Adenovirus type 7 | Adenoviru s type 55 | Human interstitial pneumonia | human metapneu movirus |
| EB virus | Measles virus | Human cytomegal ovirus | Rotavirus | Norovirus | Mumps virus | Varicella-z oster virus |
| Legionella | Bordetella pertussis | Haemophi lus influenzae | Staphylococ cus aureus | Streptococ cus pneumoni ae | Pyogenic streptococc us | Klebsiella pneumoni ae |
| Mycobacter ium tuberculosis | Mycoplasm a pneumoniae | Chlamydia pneumoni ae | Aspergillus fumigatus | Candida albicans | Candida glabrata | Cryptococ cus neoforman s |
| Cryptococc us Gattii | Coronavirus 229E | Coronavir us OC43 | Coronavirus NL63 | Coronavir us HKU1 | Coronavirus MERS | Coronavir us SARS |

### Seventh part Human genome DNA cross reaction experiment

### 1. Objective

The influence on the detection result of the 2019 novel coronavirus (2019-nCoV) nucleic acid assay kit (hybrid capture immunofluorescence method) by the human genome DNA was analyzed, and the specificity of the products was evaluated.

### 2. Material information

**Table 17 Material information required for human genome DNA cross reaction experiment**

| Serial number | Name |
|---|---|
| **1** | 2019-nCoV Throat swab sample |
| **2** | Human genome DNA |

### 3. Method

### 3.1 Source, preparation and valuation of human genome DNA

Three whole blood samples from different sources were taken, 1 mL of each of the three whole blood samples were subjected to DNA extraction by a blood genome DNA extraction system (0.1-20mL) kit of Tiangen Biotech (Beijing) Co., Ltd., and the extracted DNA was subjected to concentration test by an ultraviolet spectrophotometer.

### 3.3 Sample preparation

### Basic sample: basic sample 0.072mL+0.008mL normal saline

### Cross sample: sample 0.072mL+0.008mL DNA extracting solution

### 3.4 Measurement

The above preparation samples were detected by three batches of 2019 novel coronavirus (2019-nCoV) nucleic acid assay kits (hybrid capture immunofluorescence method) respectively, and a ratio of the cross sample detection value to the basic sample detection value was calculated.

### 4. Result

### 4.1 DNA extracting solution concentration

**Table 18 DNA extracting solution concentration**

| | DNA extracting solution 1 | DNA extracting solution 2 | DNA extracting solution 3 |
|---|---|---|---|
| Concentration | 95µg/mL | 90µg/mL | 102µg/mL |
| Volume | 0.1mL | 0.1mL | 0.1mL |

### 4.2 Throat swab sample cross result

**Table 19 Reagent cross experiment detection result (critically positive throat swab sample 1)**

| | 401200101 | | |
|---|---|---|---|
| | Basic value | Interference value | Ratio |
| DNA extracting solution 1 | 118.10 | 112.42 | 0.95 |
| DNA extracting solution 2 | 116.28 | 107.06 | 0.92 |
| DNA extracting solution 3 | 111.66 | 110.98 | 0.99 |

### 5. Conclusion

In conclusion, the detection results of the cross sample prepared by the above sample and the corresponding basic sample are consistent, and a ratio of the cross sample detection value to the basic sample detection value is between 0.9 and 1.1, indicating that there is no cross phenomenon between the reagent and the following common respiratory pathogens.

The above description shows and describes the preferred embodiments of the invention, it should be understood that the invention is not limited to the form disclosed herein and should not be regarded as an exclusion of other embodiments, but may be applied to various other combinations, modifications and environments and can be modified by the above teaching or technology or knowledge in the related field within the scope of the invention concept. However, the modifications and changes made by those skilled in the art do not depart from the spirit and scope of the invention and should fall within the protection scope of the appended Claims of the invention.

## Claims

1. A corona virus disease 2019 nucleic acid rapid hybrid capture immunofluorescence detection kit, comprising COVID-19 reaction liquid, wherein the COVID-19 reaction liquid is prepared from a COVID-19 fluorescence marker and a COVID-19 probe solution; the COVID-19 probe solution comprises: an ORF1ab segment probe, an N segment probe and an E segment probe; and the ORF1ab segment probe is used for detecting an open reading coding frame lab of the COVID-19, the N segment probe is used for detecting an envelope protein gene of the COVID-19, and the E segment probe is used for detecting a core-shell protein gene of the COVID-19.

2. The corona virus disease 2019 nucleic acid rapid hybrid capture immunofluorescence detection kit according to Claim 1, wherein
a sequence of the ORF1ab segment probe is: 
Aacgattgtgcatcagctgactgaagcatgggttcgcggagttgatcacaactacagccataacctttccacataccgcaga
c;
a sequence of the N segment probe is: Agagcagcatcaccgccattgccagccattctagcaggagaagttc; and
a sequence of the E segment probe is:
aaggatggctagtgtaactagcaagaataccacgaaagcaagaaaaa.

3. The corona virus disease 2019 nucleic acid rapid hybrid capture immunofluorescence detection kit according to Claim 1, wherein the COVID-19 fluorescence marker is prepared by coupling a fluorescent material and a COVID-19 marking raw material; the COVID-19 marking raw material adopts a COVID-19 antigen or antibody; and the fluorescent material adopts any one of the followings: a FITC fluorescein, a fluorescent microsphere, a fluorescent particle and a biological fluorescein.

4. The corona virus disease 2019 nucleic acid rapid hybrid capture immunofluorescence detection kit according to Claim 1, further comprising a COVID-19 negative reference substance, wherein the negative reference substance comprises one or more of the following reference substances: a normal saline reference substance, a purified water reference substance, a non-COVID-19 pathogen reference substance and a pseudovirus not containing a COVID-19 target sequence.

5. The corona virus disease 2019 nucleic acid rapid hybrid capture immunofluorescence detection kit according to Claim 4, wherein the negative reference substance comprises N1-N17: N1-N2 are normal saline reference substances, N3-N4 are purified water reference substances, N5-N13 are human throat swab samples and N14-N17 are pseudoviruses not containing COVID-19 target sequences; in the human throat swab samples, COVID-19 is negative and the non-COVID-19 pathogen reference substance is positive; and the pseudoviruses not containing the COVID-19 target sequences are subtypes of coronaviruses, N14 being positive for a human coronavirus 229E N segment, N15 being positive for a human coronavirus NL63 N segment, N16 being positive for a human coronavirus OC43 N segment, and N17 being positive for a human coronavirus HKU1 N segment.

6. The corona virus disease 2019 nucleic acid rapid hybrid capture immunofluorescence detection kit according to Claim 1, further comprising a COVID-19 positive reference substance, wherein the positive reference substance is a pseudovirus containing a COVID-19 target sequence; the positive control reference substance comprises P1, P2 and P3, P1 being positive for a COVID-19 N segment, P2 being positive for a COVID-19 E segment, and P3 being positive for a COVID-19 ORF 1ab segment; and a concentration of the positive reference substance is 3000 TU/mL±5%.

7. The corona virus disease 2019 nucleic acid rapid hybrid capture immunofluorescence detection kit according to Claim 1, further comprising a precision reference substance and a limit of detection reference substance, wherein the precision reference substance comprises J1, J2 and J3, J1-J2 being pseudoviruses containing COVID-19 target sequences and being positive for a COVID-19 ORF lab segment, concentrations of J1-J2 being 2000TU/mL±5% and 5000TU/mL±5% respectively, J3 being a mixed human negative throat swab sample and being negative for COVID-19; and the limit of detection reference substance comprises L1, L2 and L3, L1 being positive for a COVID-19 N segment, L2 being positive for a COVID-19 E segment, L3 being positive for a COVID-19 ORF lab segment, and a concentration of the limit of detection reference substance being 1000 TU/mL±5%.

8. The corona virus disease 2019 nucleic acid rapid hybrid capture immunofluorescence detection kit according to Claim 1, further comprising a COVID-19 detection strip, COVID-19 redissolving liquid and COVID-19 sample preserving liquid.

9. A preparation method of the corona virus disease 2019 nucleic acid rapid hybrid capture immunofluorescence detection kit according to any one of Claims 1 to 8, comprising a preparation step of the COVID-19 fluorescence marker as follows:
activation: adding 1% fluorescent microsphere solution with a ratio of 1.0mg/mL±5% and an EDC solution with a ratio of 0.6mg/mL±5% into a prepared borate buffer solution of 0.05M±5%, mixing uniformly, placing the mixture on a rotary mixer to rotate for more than 20 minutes, perform centrifugation at 15000-16000rpm for more than 30 minutes after activating, removing a supernatant, performing resuspension by the borate buffer solution of 0.05M±5% and mixing uniformly;
coupling: adding a COVID-19 antibody in an amount of 0.2mg/mL±5% into the activated fluorescent microsphere solution, mixing uniformly, and placing the mixture on the rotary mixer to rotate for more than 2 hours to obtain a fluorescent microsphere marking conjugate solution;
closing: adding a 10% BSA solution with a ratio of 0.1ml/mL±5% into the fluorescent microsphere marking conjugate solution, mixing uniformly, and placing the mixture on the rotary mixer to rotate for 12-16 hours; and
centrifugal resuspension: centrifuging the fluorescent microsphere marking conjugate solution at 15000-16000rpm, removing a supernatant, washing with an isovolumetric borate buffer solution of 0.05M±5%, and finally resuspending a precipitate with a marker diluent in a volume which is equal to that of the supernatant solution to prepare the COVID-19 fluorescence marker.

10. The preparation method according to Claim 9, comprising a preparation step of COVID-19 reaction liquid as follows:
preparation of a fluorescence marker: diluting the COVID-19 fluorescence marker with a marker diluent according to a ratio, wherein the ratio of the diluent to a T line marker to a C line marker is equal to 17:2:1;
preparation of a probe solution: diluting a COVID-19 probe with nucleic acid solving liquid to 10µM±5% to obtain a COVID-19 probe solution;
preparation of reaction liquid: mixing the diluted COVID-19 fluorescence marker and the COVID-19 probe solution according to a volume ratio of 1:1 to obtain COVID-19 reaction liquid; and
subpackaging of the reaction liquid: subpackaging the COVID-19 reaction liquid into reaction tubes and drying for 6 to 8 hours under the conditions that the temperature is 18-28°C and the humidity is less than or equal to 30%.

11. The preparation method according to Claim 9, further comprising a preparation step of a COVID-19 positive reference substance: diluting artificially synthesized COVID-19 RNA with a positive reference substance diluent to 2000TU/mL±5%; and subpackaging the diluted positive reference substances into vertical tubes and drying for 6 to 8 hours under the conditions that the temperature is 18-28°C and the humidity is less than or equal to 30%.

12. A detection method of the corona virus disease 2019 nucleic acid rapid hybrid capture immunofluorescence detection kit according to any one of Claims 1 to 8, comprising the following steps:
acquiring a target nucleic acid fragment in a to-be-detected sample through hybrid capture;
performing nucleic acid hybridization reaction on the target nucleic acid fragment and the COVID-19 reaction liquid to obtain to-be-detected liquid; and
adding the to-be-detected liquid into a COVID-19 detection strip for fluorescence signal recognition.
